Europäisches Patentamt

European Patent Office

Office européen des brevets

(11) Numéro de publication: **0 297 979**
**A2**

## (12) DEMANDE DE BREVET EUROPEEN

(21) Numéro de dépôt: **88401663.5**

(22) Date de dépôt: **29.06.88**

(51) Int. Cl.⁴: **A 61 B 5/02**

(30) Priorité: **01.07.87 FR 8709313**

(43) Date de publication de la demande:
**04.01.89 Bulletin 89/01**

(84) Etats contractants désignés:
**AT BE CH DE ES GB GR IT LI LU NL SE**

(71) Demandeur: **Ascher, Gilles**
**36, rue de la Ferme**
**F-92200 Neuilly Sur Seine (FR)**

(72) Inventeur: **Ascher, Gilles**
**36, rue de la Ferme**
**F-92200 Neuilly Sur Seine (FR)**

(74) Mandataire: **Rodhain, Claude et al**
**Cabinet Claude Rodhain 30, rue la Boétie**
**F-75008 Paris (FR)**

(54) Brassard pour sphygmomanomètre ambulatoire.

㉗ L'invention concerne un brassard pour sphygmomanomètre à mesure automatique, comportant deux bandes (2, 3) de
tissu fixées l'une sur l'autre entre lesquelles est logée une
enveloppe gonflable (16), la bande interne (2) étant munie sur
sa face intérieure d'une poche (9) dans laquelle est logée un
capteur (2) tel qu'un microphone.

Le brassard selon l'invention comporte, sur la face extérieure
(12) à la bande interne (2), dans la zone de ladite poche (9), au
moins un dispositif d'accrochage (18, 19) pour la fixation
amovible d'une pastille adhésive sur le corps humain.

L'invention s'applique en particulier au sphygmomanomètre
à usage ambulatoire.

FIG. 3

EP 0 297 979 A2

**Description**

<center>Brassard pour sphygmomanomètre ambulatoire.</center>

La présente invention concerne les sphygmomanomètres à usage ambulatoire qui sont utilisés pour mesurer de manière automatique et à intervalles réguliers la tension artérielle de patients pendant des durées importantes pouvant aller jusqu'à plusieurs jours.

Ces sphygmomanomètres à usage ambulatoire sont de type classique et constitués par un brassard qui comporte deux bandes de tissu fixées l'une sur l'autre, et entre lesquelles est logée une enveloppe gonflable ; la bande interne de ce brassard est munie, sur sa face intérieure, d'une poche dans laquelle est logée un capteur tel qu'un microphone qui doit se trouver en regard de l'artère brachiale. Cet appareil fait des mesures à intervalles réguliers, de manière automatique, de la tension artérielle du patient pendant que ce dernier a une activité normale.

Les sphygmomanomètres ambulatoires actuellement connus présentent l'inconvénient qu'il est difficile de maintenir le microphone bien en face de l'artère brachiale, en effet le brassard peut se déplacer, soit verticalement le long du bras, soit en rotation autour de ce dernier. En effet, lorsque l'on n'effectue pas de mesure, il faut que le brassard n'exerce aucune contrainte pour le patient qui doit pouvoir garder sa liberté de mouvements ; en particulier, on doit éviter toute possibilité de thrombose.

Pour éviter le déplacement du microphone capteur, on peut utiliser des bandes adhésives ; mais ce procédé est assez peu efficace et constitue une gêne pour le patient.

La présente invention a pour objet un brassard pour sphygmomanomètre ambulatoire du type précité qui permet de maintenir constamment le microphone capteur bien en place en vis à vis de l'artère brachiale.

Le brassard selon l'invention est notamment remarquable en ce qu'il comporte sur la face extérieure de la bande interne, dans la zone de ladite poche contenant le capteur, au moins un dispositif d'accrochage pour la fixation amovible d'une pastille adhésive sur le corps humain. De préférence, on utilise des pastilles amovibles du type jetable, c'est à dire qu'elles ne sont utilisées qu'une seule fois et que l'on fixe sur le dispositif d'accrochage. De cette manière, on obtient un bon positionnement constant du brassard au niveau de la poche du capteur, si bien que ce dernier reste toujours dans la bonne position par rapport à l'artère brachiale.

Le brassard peut comporter deux dispositifs d'accrochage disposés sur les deux bords de la bande interne, l'un d'entre eux se trouvant au voisinage de la poche du microphone capteur ; on peut également prévoir trois dispositifs d'accrochage, voire deux disposés de part et d'autre de la poche du microphone et un autre sur le bord opposé de la bande interne.

Selon une autre caractéristique de l'invention, on prévoit un renforcement de la poche du microphone qui est constitué par une feuille en matière synthétique fixée sur la face intérieure de la bande interne au niveau de ladite poche.

Les dispositifs d'accrochage sont avantageusement des dispositifs à clipsage ; ils peuvent être fixés à travers le renforcement précité de la poche de manière à constituer en ensemble relativement rigide de la poche du microphone et de ces dispositifs d'accrochage.

L'invention a également pour objet un brassard muni de pastilles adhésives sur le corps humain, en particulier des pastilles adhésives jetables c'est à dire que l'on n'utilise qu'une seule fois.

D'autres caractéristiques et avantages de l'invention ressortiront de la description qui suit ainsi que du dessin ci-annexé sur lequel :

    - la figure 1 est une vue du brassard brassard constituant le sphygmomanomètre selon l'invention du côté de sa face interne,

    - la figure 2 est une vue du même brassard du côté de sa face externe, et

    - la figure 3 est une coupe du brassard des figures 1 et 2, au niveau de la poche du microphone.

L'invention concerne un sphygmomanomètre ambulatoire qui est constitué par un brassard 1 comportant deux bandes en tissu cousues l'une sur l'autre, une bande interne 2 et une bande externe 3 ; la bande interne ne s'étand que sur une partie seulement de la bande externe ; la face intérieure 4 de la bande externe 3 présente dans sa partie 5 où elle n'est pas recouverte par la bande interne 2, un dispositif d'accrochage sur sa face extérieure 6 qui est également munie de dispositifs d'accrochage correspondants. Ces dispositifs d'accrochage peuvent être constitués par deux rubans à boucles et crochets pour fermeture rapide 7, respectivement 8.

Une poche 9 est cousue sur la face intérieure de la bande interne 2 ; cette poche débouche sur une ouverture 11 pratiquée dans la face extérieure 12 de la bande interne 2 ; cette ouverture 11 est située à environ 1/3 du bord inférieur et permet de sortir le microphone pour, dans une autre configuration, l'appliquer directement sur l'artère brachiale à l'aide d'une pastille adhésive. La poche 9 est prolongée par une poche longitudinale 13 constituant un conduit pour un fil de raccordement 14 du microphone qui est par exemple muni d'une fiche 15.

Entre les deux bandes interne 2 et externe 3 qui sont cousues l'une sur l'autre, sur leurs bords, se trouve une enveloppe gonflable 16 qui comporte un embout de gonflage 17 qui sort par une ouverture pratiquée entre les deux bandes 2 et 3.

Conformément à l'invention, on prévoit sur la face extérieure 12 de la bande interne 2 des dispositifs d'accrochage destinés à la fixation amovible des pastilles adhésives sur le corps humain. Cette pastille peut, par exemple, être constituée par des pastilles de type jetable qui sont utilisées une seule fois et qui sont collées sur le corps humain. On peut avantageusement prévoir un premier dispositif d'accrochage au niveau de la poche 9 et un deuxième sur le bord opposé de la bande interne 2. Dans le mode de réalisation représenté, on a prévu trois dispositifs d'accrochage ; deux dispositifs d'accrochage 18 qui sont disposés de part et d'autre de la poche 9 et un dispositif d'accrochage 19 qui est disposé le long du bord opposé de la bande interne 2.

Avantageusement, ces dispositifs d'accrochage sont du type à clipsage tel que les parties mâles que l'on utilise dans les boutons-pressions.

Selon une autre caractéristique importante de l'invention, on prévoit un renforcement de la poche de capteur, il est constitué par une feuille en matière relativement rigide telle que une matière synthétique qui est fixée sur la paroi intérieure de la bande interne au niveau de ladite poche.

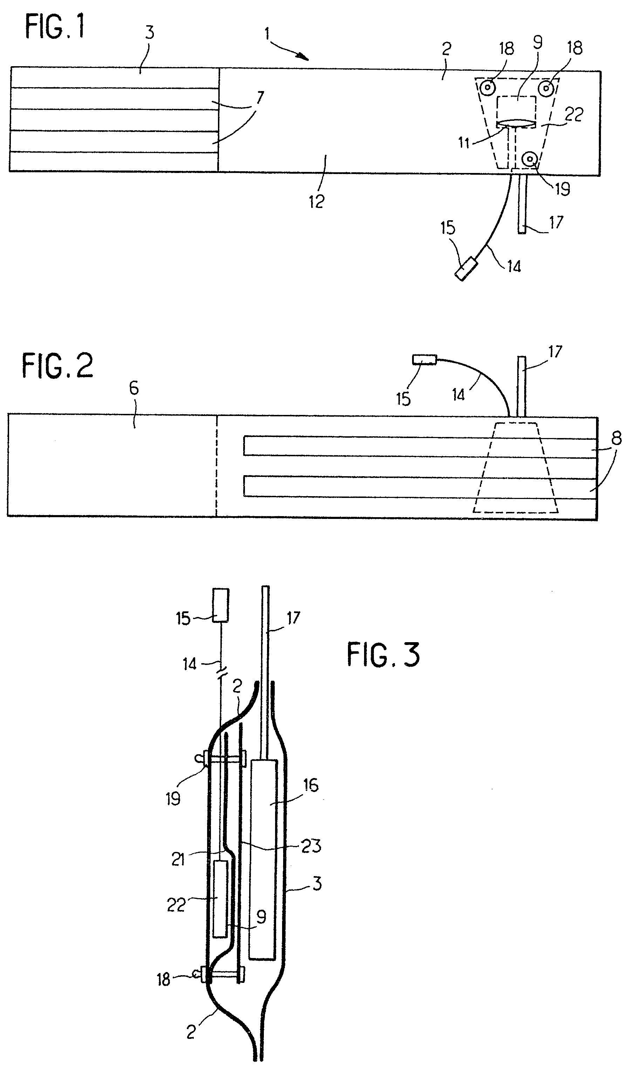

Sur la figure 3 qui est une vue en coupe transversale du brassard au niveau de la poche de capteur 9, on peut voir successivement, en partant de l'intérieur du brassard vers l'extérieur, la bande interne 2, une bande de tissu 21 formant la poche 9 dans laquelle est disposé le capteur tel qu'un microphone à électret 22, la feuille de renforcement 23 qui peut par exemple être en matière synthétique telle que celle distribuée commercialement sous le nom de "mylar", l'enveloppe gonflable 16 et la bande externe 3. Avantageusement, les dispositifs d'accrochage sont fixés sur la feuille de renforcement 23 ; ils peuvent par exemple être rivetés sur l'ensemble des bandes 2 et 21 et de la feuille de renforcement 23.

De cette manière, on réalise au niveau de la poche de capteur 9, un ensemble rigide qui comporte en particulier la poche de capteur et qui sera fixé sans possibilité de mouvement sur le patient grâce aux pastilles adhésives qui seront fixées sur les dispositifs d'accrochage 18 et 19.

L'invention permet donc d'obtenir un sphygmomanomètre ambulatoire qui permet d'effectuer des mesures sur une longue période avec une bonne fiabilité sans créer de gêne pour le patient.

## Revendications

1) Brassard pour sphygmomanomètre à mesure automatique, destiné en particulier à un usage ambulatoire, comportant deux bandes de tissu (2, 3) fixées l'une sur l'autre entre lesquelles est logée une enveloppe gonflable (16), la bande interne (2) étant munie, sur sa face intérieure, d'une poche (9) dans laquelle est logé un capteur (22) tel qu'un microphone, caractérisé en ce qu'il comporte, sur la face extérieure (12) de la bande interne (2), dans la zone de ladite poche (9), au moins un dispositif d'accrochage (18, 19) pour la fixation amovible d'une pastille adhésive sur le corps humain.

2) Brassard selon la revendication 1, caractérisé en ce qu'il comporte deux dispositifs d'accrochage, un, disposé au voisinage de ladite poche (9) et un autre disposé sur le bord opposé de la bande interne (2).

3) Brassard selon la revendication 1, caractérisé en ce qu'il comporte deux dispositifs d'accrochage (18) disposés de part et d'autre de la poche (19) et un dispositif d'accrochage (19) disposé au voisinage du bord opposé de la bande interne (2).

4) Brassard selon la revendication 1, caractérisé en ce qu'il comporte un renforcement (22) de ladite poche (9), constitué par une feuille en matière synthétique fixée sur la face intérieure de la bande interne (2) au niveau de ladite poche (9).

5) Brassard selon l'une quelconque des revendications 1 à 4, caractérisé en ce que les deux dispositifs d'accrochage (18, 19) sont des dispositifs à fixation par clipsage.

6) Brassard selon les revendications 4 et 5, caractérisé en ce que les dispositifs à clipsage (18, 19) sont fixés à travers le renforcement (22) de la poche (9).

7) Brassard selon l'une quelconque des revendications 1 à 6, muni de pastilles adhésives sur le corps humain du type jetable.

8) Brassard selon l'une quelconque des revendications 1 à 7, caractérisé en ce que la poche (9) débouche sur une ouverture (11) pratiquée dans la face extérieure (12) de la bande interne (2), ladite ouverture permettant de sortir le microphone et de le fixer sur le bras à l'aide d'une pastille adhésive.

FIG.1

FIG.2

FIG.3